# EUROPEAN PATENT APPLICATION

(11) **EP 4 306 145 A1**
(43) Date of publication of application: **17.01.2024**
(21) Application number: 22187240.1
(22) Date of filing: 27.07.2022
(51) Int. Cl.: A61M 5/142

(54) **MEDICAL INFUSION DEVICE COMPRISING A LEAKAGE DETERMINING MODULE**

(30) Priority: 13.07.2022 US 202263388770 P
(71) Applicant: F. Hoffmann-La Roche AG, 4070 Basel (CH); Roche Diabetes Care GmbH, 68305 Mannheim (DE)
(72) Inventor: SMITH, Aaron, Indianapolis 46250, Indiana (US); FERNSNER, Stefan, 68305 Mannheim (DE); JANKE, Christian, 68305 Mannheim (DE)
(74) Representative: Pfaffmann, Lucas

(57) **Abstract**

The present invention relates to a medical infusion device comprising a reservoir for holding a liquid medicament, a cannula assembly, a fluid line, a drive mechanism, a dispensing member for dispensing medicament from reservoir through the fluid line and the cannula assembly, and a leakage determining module. For a more convenient determination of leakage of liquid medicament, the leakage determining module comprises an electronic probe that is configured for receiving odor molecules and/or flavor molecules of the liquid medicament and in that the leakage determining module further comprises evaluation electronics that is configured to determine presence of odor and/or flavor of the liquid medicament based on readings of the at least one electronic probe.

## Description

The present invention relates to a medical infusion device comprising a reservoir that is configured for holding a liquid medicament, a cannula assembly, a fluid line that is in fluid communication with the reservoir and the cannula assembly, a drive mechanism, a dispensing member that when being actuated by the drive mechanism dispenses liquid medicament out of the reservoir through the fluid line and the cannula assembly, and a leakage determining module that is configured for determining leakage of the liquid medicament out of at least one of the reservoir, the fluid line or the cannula assembly.

Medical infusion devices are used for providing a user of the infusion device with a liquid medicament that is intended to improve the patient's health condition. Therefore, liquid medicament that is contained within the reservoir of the medical infusion device is dispensed through the fluid line and the cannula assembly by a dispensing member that is actuated by a drive mechanism. However, sometimes a malfunction occurs that results in an unintended leakage of liquid medicament out of one or more of the reservoir, the fluid line or the cannula assembly. For instance, a leakage can be caused by a mechanical impact on the medical infusion device, in particular mechanical impact on the liquid medicament containing or guiding components, or an improper assembly of the medical infusion device.

A leakage may result in not providing the desired amount of liquid medicament to the user of the medical infusion device. Often a leakage, in particular of small amounts of liquid medicament will not be noticed by the user. This may result in an underdosing or even overdosing of liquid medicament. The latter may occur in case the desired effect of the liquid medicament is missing and the medical infusion device is operated to compensate a leakage of liquid medicament, but the amount of the under dosed liquid medicament actually provided is unknown to the user or to a semi-/fully automated delivery algorithm of the medical infusion pump, if the medical infusion pump is operated with such an algorithm.

Therefore, a hitherto known medical infusion device comprises a leakage determining module that is configured for determining leakage of the liquid medicament out of at least one of the reservoir, the fluid line or the cannula assembly. However, those leakage determining module of a hitherto known medical infusion pump is an indicator paper or a humidity sensor. Use of an indicator paper is cumbersome for a user of the medical infusion pump as the indication of a leakage is only apparent by frequently looking at the indicator paper. Furthermore, the indicator paper is unspecific to the type of liquid wetting the indicator paper. Thus, depending on the user's environment the indicator paper falsely indicates a leakage of liquid medicament due to a water or humidity ingress. This is also true when a humidity sensor is used instead of an indicator paper.

Accordingly, it is an object of the present invention to provide a medical infusion device that avoids at least one of the problems associated with medical devices known from the prior art. In particular, it is an object of the invention to provide a medical infusion device which allows for a more convenient determination of leakage of liquid medicament.

At least one of the above objects is solved by the provision of the subject-matter disclosed in the claims and throughout the specification.

According to a first aspect, a medical infusion device is provided. The medical infusion device comprises a reservoir that is configured for holding a liquid medicament, a cannula assembly, a fluid line that is in fluid communication with the reservoir and the cannula assembly, a drive mechanism, a dispensing member that when being actuated by the drive mechanism dispenses liquid medicament out of the reservoir through the fluid line and the cannula assembly, and a leakage determining module that is configured for determining leakage of the liquid medicament out of at least one of the reservoir, the fluid line or the cannula assembly, wherein the leakage determining module comprises an electronic probe that is configured for receiving odor molecules and/or flavor molecules of the liquid medicament and in that the leakage determining module further comprises evaluation electronics that is configured to determine presence of odor and/or flavor of the liquid medicament based on readings of the at least one electronic probe.

During use, liquid medicament is contained in the reservoir of the medical infusion device, wherein upon actuating the drive mechanism the dispensing member forces liquid medicament to flow out of the reservoir into the fluid line and finally through the cannula assembly. A leakage of liquid medicament is any non-intended flow of liquid medicament out of one of the reservoir, the fluid line or cannula assembly. The cannula assembly can comprise a septum and a connection needle that during use pierces the septum in order to provide a fluid communication from the reservoir via the fluid line via the connecting line through the septum to the cannula.

Liquid medicament leaking out of at least one of the reservoir, the fluid line and the cannula assembly is determined by the leakage determining module. The leakage determining module is configured to detect odor and/or flavor of the liquid medicament. The leakage determining module comprises an electronic probe that is configured to receive odor molecules and/or flavor molecules of the liquid medicament. I.e. odor molecules and/or flavor molecules of the liquid medicament leaking out of at least one of the reservoir, the fluid line or the cannula assembly are received by the electronic probe, wherein upon receipt of the odor molecules and/or flavor molecules of the liquid medicament an electronic signal is generated by the electronic probe.

The electronic signal generated by the electronic probe, i.e. the reading of the electronic probe, is received by the evaluation electronics of the leakage determining module. The evaluation electronics determine presence of odor molecules and/or flavor molecules based on the reading of the electronic probe. The electronic probe and the evaluation electronics together form an electronic sensing device that is configured to detect odor and/or flavor of the liquid medicament.

According to an embodiment of the present invention, the electronic probe comprises at least one of a metal-oxide-semiconductor ("MOSFET"), conducting polymers, non-conducting polymers with the addition of conducting material, such as carbon black, quartz crystal microbalance ("QCM"), surface acoustic wave ("SAW'), and mass spectrometers.

In the meaning of the present invention, a metal-oxide-semiconductor is a transistor that amplifies or switches electronic signals. Odor molecules and/or flavor molecules received by the probe will be charged either positively or negatively thereby changing the electric field inside the MOSFET. Odor molecules and/or flavor molecules will change the MOSFET signal than can be interpreted by the evaluation electronics.

In the meaning of the present invention, conducting polymers are organic polymers that conduct electricity, wherein upon receiving odor molecules and/or flavor molecules the conductivity of the polymers respectively change thereby generating a sensor signal that can be interpreted by the evaluation electronics.

Likewise, in the meaning of the present invention, non-conducting polymers with the addition of conducting material, such as for example carbon black, will also change the conductivity when receiving odor molecules and/or flavor molecules. The change in conductivity leads to a change in the sensor signal that can be interpreted by the evaluation electronics.

In the meaning of the present invention, a quartz crystal microbalance measures a mass variation per unit area by measuring the change in frequency of a quartz crystal resonator. Upon receiving odor molecules and/or flavor molecules the mass per unit area is varied, which will result in a change in frequency of the quartz crystal resonator. The change in frequency generates a sensor signal that can be interpreted by the evaluation electronics.

Further, in the meaning of the present invention a surface acoustic wave is a microelectromechanical system, which rely on the modulation of surface acoustic waves to sense a deposition of odor molecules and/or flavor molecules. For example, an acoustic wave is travelling along the surface of an elastic material with an amplitude that decays exponentially with depth into the material. Upon receiving odor molecules and/or flavor molecules the physical properties of the micromechanical system changes which affects the surface acoustic wave. Thus, a sensor signal generated on basis of the change in the surface acoustic wave can be interpreted by the evaluation electronics to determine presence of odor molecules and/or flavor molecules.

In the meaning of the present invention, evaluation electronics comprises hardware and/or software that is configured to interpret the readings of the at least one electronic probe and/or an electronic reference probe. For example, the evaluation electronics can be a microprocessor or part thereof. According to an embodiment, the evaluation electronics for interpreting the readings of the at least one electronic probe and/or the at least one electronic reference probe is configured for pattern recognition. According to an embodiment, the evaluation electronics for interpreting the readings of the at least one electronic probe and/or the at least one electronic reference probe can be trained by using a neural network.

According to an embodiment of the present invention, the at least one electronic probe is located at one or more locations on or at the outside of the reservoir, and/or at one or more locations on or at the outside of the cannula assembly, and/or at one or more locations on or at the outside of the fluid line. Once the liquid medicament has leaked out of one of the reservoir, the fluid line or the cannula assembly, the direction of the flow of liquid medicament is unknown.

According to an embodiment of the present invention, the medical infusion device comprises at least one drain channel that is configured for guiding liquid medicament that is leaking from at least one of the reservoir, the fluid line or the cannula assembly towards the at least one electronic probe of the leakage determining module. The at least one drain channel directs liquid medicament leaking out of at least one of the reservoir, the fluid line, or the cannula assembly towards the at least one probe. Thereby, the at least one probe can be arranged at any location within the medical infusion device. In particular, the at least one probe can be arranged at a location away from the reservoir, the fluid line or the cannula assembly. The at least one probe can be located at a location distinct from a location that is prone to spring a leak, e.g. the transition between the reservoir and the fluid line, the transition between the fluid line and the cannula assembly, an inlet of the reservoir, if present, or an outlet of the reservoir. Furthermore, the medical infusion device can comprise multiple drain channels each configured for guiding liquid medicament that is leaking from at least one of the reservoir, the fluid line or the cannula assembly towards the at least one probe. Thereby at least one probe can be used to detect liquid medicament that may leak out of different locations of at least one of the reservoir, the fluid line or the cannula assembly.

According to an embodiment of the present invention, the medical infusion device comprises at least one drain chamber that is configured for collecting liquid medicament that is leaking out of at least one of the reservoir, fluid line or cannula assembly, wherein the drain chamber comprises the at least one electronic probe of the leakage determining module. According to yet another embodiment, the at least one drain chamber is in fluid communication with the at least one drain channel. Liquid medicament collected in the drain chamber is guided through the drain channel from the drain chamber towards the electronic probe or the at least one drain channel guides liquid medicament leaking out of at least one of the reservoir, the fluid line or the cannula assembly into the at least one drain chamber, wherein the drain chamber comprises the at least one electronic probe of the leakage determining module. Thus, instead of arranging the at least one electronic probe at a location or near a location which is prone spring leakage, the at least one probe is arranged at a location within a guiding system comprising the at least one drain channel and/or the at least one drain chamber. This allows a more flexible configuration of the medical infusion device, which is in particular beneficial with regards of a desired shape factor to be obtained by the medical infusion device.

According to an embodiment of the present invention, the at least one electronic probe is located at one or more locations on or at the outside of the medical infusion device, wherein the at least one electronic probe is configured and located to receive odor molecules and/or flavor molecules at the outside of the medical infusion device. By way of example, only, the at least one electronic probe can be integrated in or located at a housing of the medical infusion device. Liquid medicament leaking from at least one of the reservoir, the fluid line or the cannula assembly may somehow flow out of the medical infusion device along the periphery of the medical infusion device or, during use of the medical infusion device, may accumulate outside of the medical infusion device. For instance, between the medical infusion device and a patient's body, or in the vicinity thereof.

According to an embodiment of the present invention, the leakage determining module comprises at least one electronic reference probe that is configured and arranged for receiving odor molecules and/or flavor molecules of the liquid medicament outside of the medical infusion device and wherein the evaluation electronics is further configured to determine presence of odor and/or flavor of the liquid medicament based on readings of the at least one electronic reference probe and the readings of the at least one electronic probe. During use of the medical infusion device, liquid medicament can be present outside of the medical infusion device without the need of a leakage of at least one of the reservoir, the fluid line or the cannula assembly, for instance due to a previous leakage, mishandling or contamination. The reference probe provides a reading of odor molecules and/or flavor molecules of the liquid medicament that might be present on the outside of the medical infusion device. The evaluation electronics based on the readings of the electronic probe and the electronic reference probe determines whether liquid medicament is leaking out of at least one of the reservoir, the fluid line, or the cannula assembly. For instance, in case the reading of the electronic probe is indicative of a presence of odor molecules and/or flavor molecules of the liquid medicament, while the reading of the electronic reference probe does not indicate presence of odor molecules and/or flavor molecules the evaluation electronics determines that there is a leakage of liquid medicament out of at least one of the reservoir, the fluid line, or the cannula assembly. Furthermore, for example in case both readings of the at least one electronic probe and the at least one electronic reference probe are indicative of a presence of odor molecules and/or flavor molecules, the evaluation electronics determines that there is no liquid medicament leaking out of at least one of the reservoir, the fluid line or the cannula assembly. Likewise, in case for example only the readings of the at least one electronic reference probe is indicative for a presence of odor molecules and/or flavor molecules of the liquid medicament the evaluation electronics determines that there is no liquid medicament leaking out of at least one of the reservoir, the fluid line or the cannula assembly.

While the at least one electronic probe is intended to indicate presence of odor molecules and/or flavor molecules to confirm a leakage, the at least one electronic reference probe is intended to indicate absence of odor molecules and/or flavor molecules in order to prevent false positive conclusions.

According to an embodiment of the present invention, the at least one electronic probe and/or the at least one electronic reference probe, if present, are configured to receive volatile odor molecules and/or volatile flavor molecules of the liquid medicament. According to an embodiment, the at least one electronic probe and/or the at least one electronic reference probe, if present, are configured to receive gaseous odor molecules and/or gaseous flavor molecules.

According to an embodiment of the present invention, the medical infusion device comprises a user interface that is configured to indicate to the user of the medical infusion device presence of a leakage of liquid medicament based on the determination of the leakage determining module. For example, the user interface may provide a visible, audible or haptic notification to the user. The notification indicating a leakage of the medical infusion device is provided to the user, when the leakage determining module determined a leakage of liquid medicament out of at least one of the reservoir, the fluid line or the cannula assembly. According to an embodiment of the present invention, the medical infusion device is part of a medical infusion device system, wherein the user interface, that is configured to indicate to the user of the medical infusion device presence of a leakage of liquid medicament based on the determination of the leakage determining module is part of a remote control. The remote control can be a dedicated remote controller or a software application that is operated on a smartphone.

According to an embodiment of the present invention, the medical infusion device comprises a disposable portion and a reusable portion, wherein the disposable portion comprises at least the reservoir, the fluid line, the cannula assembly and at least one electronic probe of the leakage determining module and/or at least one electronic reference probe of the leakage determining module. The disposable portion is intended to be frequently more often replaced by a user as its components, like the reservoir, fluid line, and cannula assembly. The at least one electronic probe as well as the at least one electronic reference probe are intended to get in contact with odor molecules and/or flavor molecules of the liquid medicament. Thus, there may be a need for replacing the at least one electronic probe and the at least one electronic reference probe more often, which can be achieved by providing them with the disposable portion of the medical infusion device. Notwithstanding, that the at least one electronic probe and/or the at least one electronic reference probe is part of the disposable portion, the at least one electronic probe and the at least one electronic reference probe, if present, are still operatively coupled to the evaluation electronics for determining leakage of liquid medicament out of at least one of the reservoir, the fluid line, or the cannula assembly.

According to an embodiment of the present invention, the reusable portion comprises the evaluation electronics.

Consequently, both the at least one electronic probe and/or the at least one electronic reference probe as well as the evaluation electronics can be part of the disposable portion or the reusable portion, either together or separately arranged in one of the disposable portion and reusable portion. However, in any case the at least one electronic probe and the at least one electronic reference probe, if present, are always operatively coupled to the evaluation electronics.

According to an embodiment of the present invention, the disposable portion comprises the evaluation electronics.

According to an embodiment of the present invention, the leakage determining module is reversibly separable from the medical infusion device. A reversibly separately leakage determining module can be operatively coupled to and decoupled from the medical infusion device. Thereby, a worn leakage determining module can be replaced be a new leakage determining module without the need of replacing the entire medical infusion device. Likewise, in case the at least one electronic probe and/or at least one electronic reference probe and/or the evaluation electronic are separable from each other, those can be individually replaced without the need of replacing the respective other probes or evaluation electronics.

According to an embodiment of the present invention, the evaluation electronics is configured to pause determining a leakage of liquid medicament out of at least one of the reservoir, the fluid line or the cannula assembly for a predetermined period of time subsequent to a determination of a leakage of liquid medicament out of at least one of the reservoir, the fluid line or the cannula assembly. Odor molecules and/or flavor molecules reaching the at least one electronic probe or the at least one electronic reference probe, if present, can occupy an area of the respective probe that is sensitive to the odor and/or flavor molecules of the liquid medicament. Excessive occupation of the sensitive area negatively affects the sensitivity of the respective electronic probe. According to an embodiment, occupation of the sensitive area by odor molecules and/or flavor molecules can be reduced by evaporation of the received odor molecules and/or flavor molecules, self-cleaning or cleaning of the respective electronic probe, if applicable.

Furthermore, once a leakage of liquid medicament has been determined by the leakage determining module, there is no need for continually determination of a leakage until the reason for the leakage has been removed. Continually determining a presence of odor molecules and/or flavor molecules can increase the wear of the respective probe. Pausing the determination of a leakage of liquid medicament by the leakage determining module is beneficial with regards to the sensitivity and wear of the respective probe.

According to an embodiment of the present invention, duration of the pause can be limited by a predetermined time period passing by or by a user input resuming the determination of leakage of liquid medicament out of one of the reservoir, the fluid line or the cannula assembly by the leakage determination module.

In order to avoid an excessive flushing of the at least one electronic probe and/or the at least one electronic reference probe, if present, by the liquid medicament, according to an embodiment the at least one electronic probe and/or the at least one electronic reference probe, if present, comprise a gas-permeable membrane that is configured to be permeable for gaseous odor molecules and/or gaseous flavor molecules of the liquid medicament, and impermeable for liquid components of the liquid medicament. For example, the gas-permeable membrane covers an area of the electronic probe or electronic reference probe, respectively, that is sensitive for volatile odor molecules and/or volatile flavor molecules.

According to an embodiment, the medical infusion device is a body-wearable medical infusion pump, preferably a body-wearable medical patch pump. The body-wearable medical infusion pump can comprise user interface. For example, a display, a touchscreen, one or more buttons, one or more switches, a loudspeaker, one or more lights. The body-wearable medical infusion pump as well as the body-wearable medical patch pump can lack a graphical user interface on the pump, but can be operatively coupled to a remote control comprising a graphical user interface. For example, the graphical user interface can be a touchscreen. The remote control can be operatively coupled to the body-wearable medical patch pump or the body-wearable medical infusion pump by using wireless communication means. According to an embodiment, the body-wearable medical patch pump comprises a disposable portion and a reusable portion, wherein the reusable portion comprises a microprocessor, and a communications module that is configured to communicate with a remote control, such as a dedicated remote controller or a software application operating on a smartphone, and optionally a single use battery or a rechargeable battery. The disposable portion comprises the reservoir, the fluid line, and the cannula assembly. A single use or rechargeable battery can be contained in one of the disposable portion and/or the reusable portion.

According to an embodiment, the liquid medicament is insulin or a medical solution containing insulin. Accordingly, the at least one electronic probe and/or the at least one electronic reference probe, if present, is configured to receive odor molecules and/or flavor molecules of insulin or of a medical solution containing insulin or components thereof.

Furthermore, at least one of the aforementioned objects is also solved by a medical infusion system. The medical infusion system comprising a medical infusion device and a remote control. The medical infusion device comprises a reservoir that is configured for holding a liquid medicament, a cannula assembly, a fluid line that is in fluid communication with the reservoir and the cannula assembly, a drive mechanism, and a dispensing member that when being actuated by the drive mechanism dispenses liquid medicament out of the reservoir through the fluid line and the cannula assembly. The medical infusion device is operatively coupled to the remote control, for example via a wireless communication channel. The medical infusion device, except of the presence of the leakage determining module, can be of the kind as described above.

The remote control includes a leakage determining module that is configured for determining leakage of the liquid medicament out of at least one of the reservoir, the fluid line or the cannula assembly of the medical infusion device. The leakage determining module comprises an electronic probe that is configured for receiving odor molecules and/or flavor molecules of the liquid medicament, and an evaluation electronics that is configured to determine presence of odor and/or flavor of the liquid medicament based on readings of the at least one electronic probe. The at least one electronic probe is a directional electronic probe that is configured to receive odor molecules and/or flavor molecules of the liquid medicament originating from outside of the remote control. During its use, the remote control can be moved in close proximity relative to the medical infusion device, such that the directional electronic probe can receive odor molecules and/or flavor molecules of the liquid medicament in close proximity to the medical infusion device. Thereby the user can check the medical infusion device for a leakage of the liquid medicament by sensing the medical infusion device or components thereof with the directional electronic probe of the remote control. Sensing of the medical infusion device can also help the user to target the origin of leakage and/or can further help to confirm a proper state of one or more of the medical infusion device, the reservoir, the fluid line, or the cannula assembly. For example, the user can sense the medical infusion device or the components thereof to identify or scan for micro cracks in the reservoir, fluid line or cannula assembly. The user can be notified of the result of the determination of odor molecules and/or flavor molecules by a visual, audible or haptic feedback provided by the remote control. Hence, the remote control comprises a user interface that is configured to provide a visual, acoustic or haptic notification to the user in case the leakage determining module determines presence of liquid medicament. According to an embodiment, the remote control is configured to send a signal indicative of the result of the leakage determination to a further device, for example the medical infusion device of the medical infusion system. The further device, for example the medical infusion device, can be configured to receive the corresponding signal indicative of the result of the leakage determination and to trigger a notification to a user. For instance, upon receiving a signal indicative of the result of the leakage determination, the medical infusion device may provide an acoustic, visual or haptic feedback to the user.

Insofar, as in the foregoing as well as in the following detailed description of embodiments and claims, reference is made to an electronic probe of the medical infusion device, the characteristics thereof are applicable for the directional electronic probe of the remote control of the medical infusion device, and vice versa.

Further advantages, features and technical effects of the present invention are apparent from the following description of embodiments and the accompanying figures.
- Figure 1: shows a schematic view of a medical infusion device according to an embodiment of the present invention;
- Figure 2: shows a schematic view of a medical infusion device according to another embodiment of the present invention;
- Figure 3: shows a schematic view of a medical infusion device according to another embodiment of the present invention;
- Figure 4: shows a schematic view of a medical infusion device according to yet another embodiment of the present invention;
- Figure 5: shows a schematic view of a medical infusion device according to an embodiment of the present invention; and
- Figure 6: shows a schematic view of a medical infusion system according to an embodiment of the present invention.

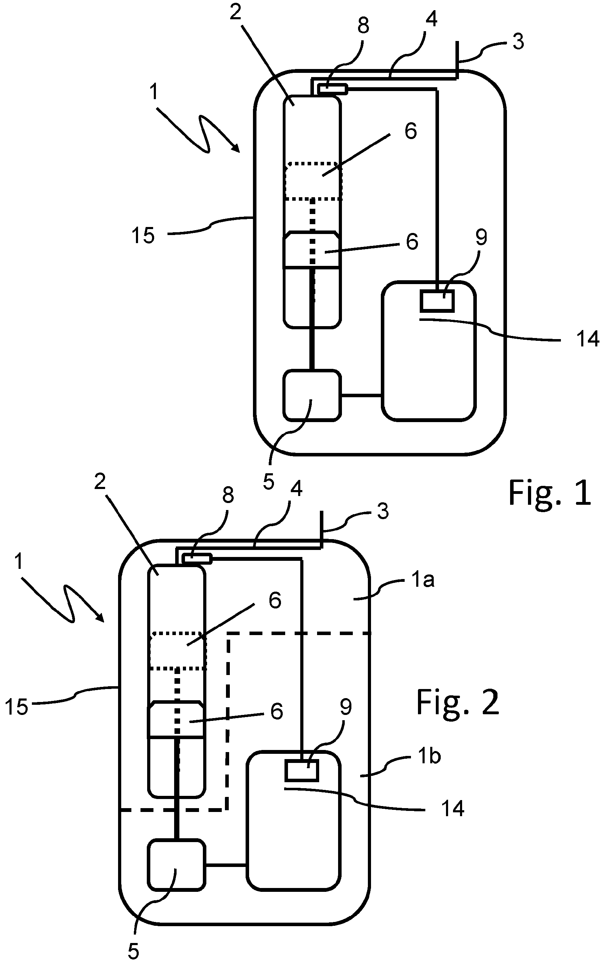

Figure 1 shows a schematic view of a medical infusion device according to an embodiment of the present invention. The medical infusion device 1 comprises a housing 15 and therein a reservoir 2 that is configured for holding a liquid medicament, such as for example insulin. The reservoir 2, thus, forms a container for the liquid medicament. The medical infusion device 1 further comprises a dispensing member 6 that is moveably arranged within the reservoir 2. When being actuated by a drive mechanism 5, the dispensing member 6 is advanced within the reservoir 2 to dispense liquid medicament out of the reservoir 2 into a fluid line 4. Movement of the dispensing member 6 is indicated in figure 1 by a broken line, by way of example, only. The fluid line 4 is fluidly coupled to a cannula assembly 3. Thus, when moving the dispensing member 6 within the reservoir 2 in a first direction liquid medicament contained in the reservoir 2 is dispensed out of the reservoir 2 into the fluid line 4 and finally through the cannula assembly 3.

The medical infusion device 1 further comprises a microprocessor 14 that is operatively coupled to the drive mechanism 5. The microprocessor 14 is configured to control operation of the drive mechanism 5.

For detecting a leakage of liquid medicament out of the reservoir 2 and/or the fluid line 4, an electronic probe 8 is arranged at the coupling between the reservoir 2 and the fluid line 4. The location of the electronic probe 8 is at the outside of the reservoir 2 and the fluid line 4, in close proximity of 5 mm or less to the outside periphery of the reservoir 2 and the fluid line 4. The electronic probe 8 is configured to receive odor molecules and/or flavor molecules of the liquid medicament. Odor molecules and/or flavor molecules of the liquid medicament leaking out of the reservoir 2 and/or the fluid line 4 will be at received by the electronic probe 8, for instance in a sensitive area thereof. The electronic probe 8, upon receiving odor molecules and/or flavor molecules of the liquid medicament will generate an electronic signal indicative of the presence of odor molecules and/or flavor molecules of the liquid medicament. The electronic signal provided with the electronic probe 8, i.e. the reading of the electronic probe 8, is transmitted to an evaluation electronics 9.

The evaluation electronics 9 is included in the microprocessor 14 and is configured to determine presence of odor and/or flavor of the liquid medicament based on the readings of the electronic probe 8. The electronic probe 8 and the evaluation electronics together form the leakage determining module 7.

Figure 2 shows a schematic view of a medical infusion device according to a second embodiment of the present invention. The medical infusion device 1 of figure 2 corresponds to the embodiment as shown in figure 1, except that the medical infusion device 1 comprises a disposable portion 1a and a reusable portion 1b. The disposable portion 1a comprises the reservoir 2, the fluid line 4, the cannula assembly 3, the dispensing member 6 and the electronic probe 8. The reusable portion 1b comprises the drive mechanism 5 and the microprocessor 14, including the evaluation electronics 9. The electronic probe 8 and the evaluation electronics 9 are operatively coupled to form the leakage determining module 7. Readings from the electronic probe 8 is transmitted to the evaluation electronics 9 for determining presence of odor and/or flavor of the liquid medicament. Disposable portion 1a and reusable portion 1b are separable so that the disposable portion 1a and the reusable portion 1b can be separately replaced as needed. Therefore, the electronic probe 8 and the evaluation electronics 9 are reversibly separable.

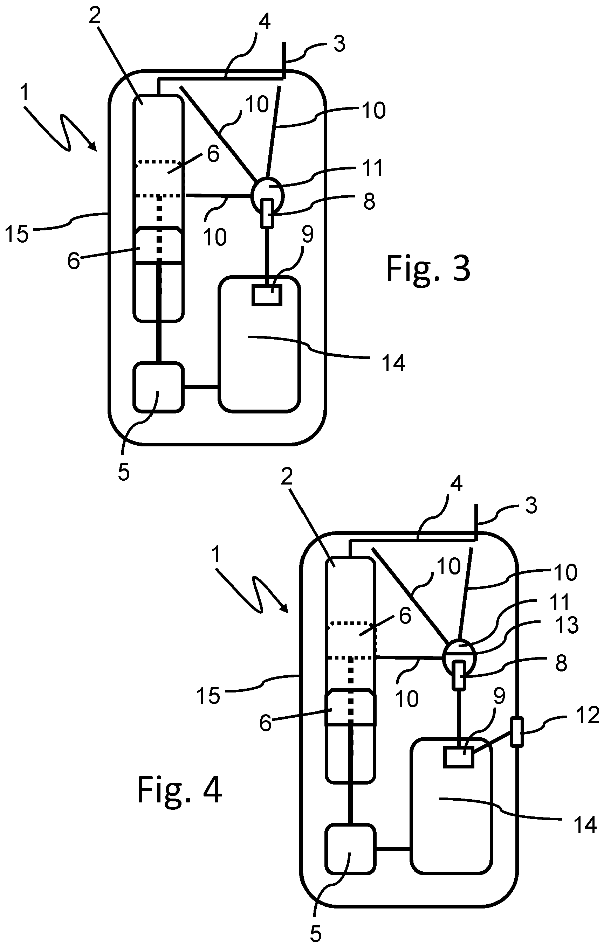

Figure 3 shows a schematic view of another embodiment of a medical infusion device 1 according to the present invention. The medical infusion device 1 as shown in figure 3 differs from the embodiment as shown in figure 1 by having three drain channels 10 and a drain chamber 11. In contrast to the embodiment shown in figure 1, the electronic probe 8 of the medical infusion device 1 of the embodiment as shown in figure 3 is arranged in the drain chamber 11. Odor molecules and/or flavor molecules of the liquid medicament leaking from one of at least the reservoir 2, the fluid line 4 or the cannula assembly 3 enter at least one of the three drain channels 10 and convey into the drain chamber 11. After entering the drain chamber 11, the odor molecules and/or flavor molecules reach the electronic probe 8, where they are received from a sensitive area of the electronic probe 8.

Figure 4 shows a schematic view of another embodiment of a medical infusion device 1 according to the present invention. The medical infusion device 1 differs from the embodiment of the medical infusion device 1 as shown in figure 3 by additionally comprising an electronic reference probe 12 and a gas-permeable membrane 13.

The gas-permeable membrane 13 is permeable for volatile odor molecules and/or flavor molecules and impermeable for liquid components of the liquid medicament. The gas-permeable membrane 13 is arranged within the drain chamber 11 and thus prevents excessive occlusion of the electronic probe 8 by liquid components of the liquid medicament.

The electronic reference probe 12 is configured and arranged for receiving odor molecules and/or flavor molecules of the liquid medicament outside of the medical infusion device 1. Here the electronic reference probe 12 is located at the periphery of the housing 15 of the medical infusion device 1. The electronic reference probe 12 is operatively coupled to the evaluation electronics 9, such that readings of the electronic reference probe 12 are transmitted to the evaluation electronics 9 for determining presence of odor molecules and/or flavor molecules of the liquid medicament. The determination of presence of liquid medicament based on the readings of the electronic reference probe 12 helps to prevent false positive determination of a leakage of liquid medicament.

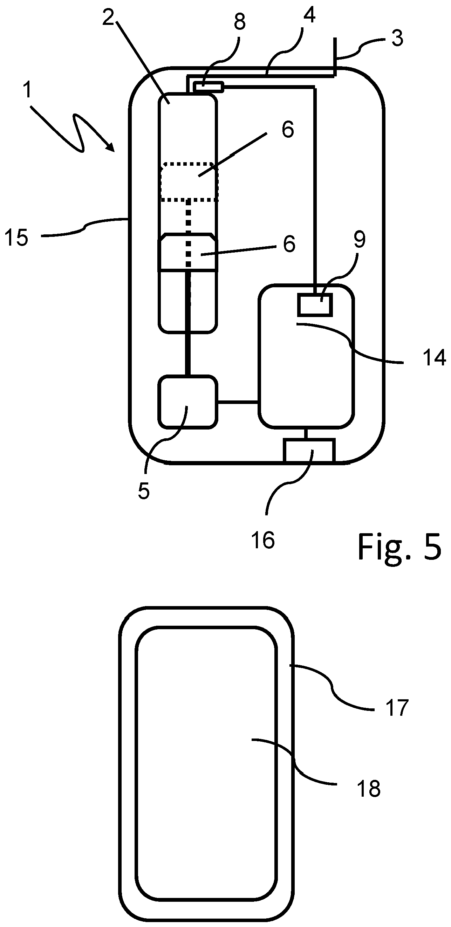

Figure 5 shows a schematic view of a medical infusion device according to an embodiment of the present invention. The medical infusion device 1 corresponds to the medical infusion device 1 of the embodiment according figure 1, but additionally comprises a wireless communications module 16. The wireless communications is operatively coupled to the microprocessor 14 and configured to communication with a remote control 17, here: a smartphone comprising a touchscreen 18. Upon determination of liquid medicament leaking out of at least one of the reservoir 2, the fluid line 4 or the cannula assembly 3 by the evaluation electronics 9 based on the readings of the electronic probe 8, an electric signal indicative of the determination is send via the wireless communications module 16 to the remote control 17. The remote control 17 notifies the user by displaying a respective notification on the touchscreen of the remote control 17.

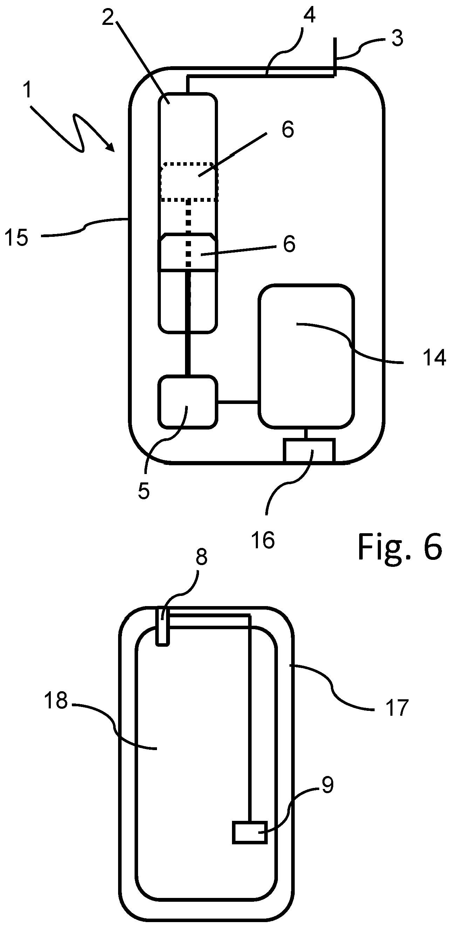

Figure 6 shows a schematic view of a medical infusion system according to an embodiment of the present invention. The medical infusion system comprises a medical infusion device 1 and a remote control 17.

The medical infusion device 1 comprises a housing containing a reservoir 2, a fluid line 4 and a cannula assembly 3. A dispensing member 6 is moveably arranged within reservoir 2, wherein the movement of the dispensing member 6 is induced by a drive mechanism 5. When advancing within the reservoir 2 liquid medicament contained in the reservoir 2 is dispensed out of the reservoir 2 into the fluid line 4 and through the cannula assembly 3.

The medical infusion device 1 further comprises a microprocessor 14 that is operatively coupled to the drive mechanism 5 and configured for controlling operation of the drive mechanism 5. Microprocessor 14 is further operatively coupled to a wireless communications module 16 for receiving and transmitting signals from and to the remote control 17.

The remote control 17, here for example a smartphone, comprises a user-interface, e.g. a touchscreen 18, a leakage determining module comprising an electronic probe 8 and evaluation electronics 9. The electronic probe 8 is configured for receiving odor molecules and/or flavor molecules of the liquid medicament, wherein the evaluation electronics 9 is configured to determine presence of odor and/or flavor of the liquid medicament based on readings of the electronic probe 8. The electronic probe 8 is a directional electronic probe that is configured to receive odor molecules and/or flavor molecules of the liquid medicament originating from outside of the remote control 17. When being used, the remote control 8 can be moved relative to the medical infusion device 1 to sense the medical infusion device 1 or components thereof with the directional electronic probe 8 of the remote control 17 in order to determine whether liquid medicament is leaking out of at least one of the reservoir 2, fluid line 4 and the cannula assembly 3.

The remote control 17 is further configured to notify the user in case the evaluation electronics 9 determines presence of a leakage of at least one of the reservoir 2, the fluid line 4 and the cannula assembly 3 based on the readings of the electronic probe 8. For example, the remote control 17 can display a notification on the touchscreen 18 of the remote control 17 in order to inform the user about the result of the determination of a leakage of liquid medicament out of at least one of the reservoir 2, the fluid line 4 and the cannula assembly 3.

### List of reference numbers

- 1: medical infusion device
- 1a: disposable portion of medical infusion device
- 1b: reusable portion of medical infusion device
- 2: reservoir
- 3: cannula assembly
- 4: fluid line
- 5: drive mechanism
- 6: dispensing member
- 7: leakage determining module
- 8: electronic probe
- 9: evaluation electronics
- 10: drain channel
- 11: drain chamber
- 12: electronic reference probe
- 13: gas-permeable membrane
- 14: microprocessor
- 15: housing
- 16: wireless communications module
- 17: remote control
- 18: touchscreen (user interface)

## Claims

1. A medical infusion device (1) comprising
• a reservoir (2) that is configured for holding a liquid medicament,
• a cannula assembly (3),
• a fluid line (4) that is in fluid communication with the reservoir (2) and the cannula assembly (3),
• a drive mechanism (5),
• a dispensing member (6) that when being actuated by the drive mechanism (5) dispenses liquid medicament out of the reservoir (2) through the fluid line (4) and the cannula assembly (3), and
• a leakage determining module (7) that is configured for determining leakage of the liquid medicament out of at least one of the reservoir (2), the fluid line (4) or the cannula assembly (3),
**characterized in that**
• the leakage determining module (7) comprises an electronic probe (8) that is configured for receiving odor molecules and/or flavor molecules of the liquid medicament and **in that** the leakage determining module (7) further comprises evaluation electronics (9) that is configured to determine presence of odor and/or flavor of the liquid medicament based on readings of the at least one electronic probe (8).

2. Medical infusion device (1) according to claim 1, **characterized in that** the at least one electronic probe (8) is located at one or more locations on or at the outside of the reservoir (2), and/or at one or more locations on or at the outside of the cannula assembly (3), and/or at one or more locations on or at the outside of the fluid line (4).

3. Medical infusion device (1) according to claim 1 or 2, **characterized in that** the medical infusion device (1) comprises at least one drain channel (10) that is configured for guiding liquid medicament that is leaking from at least one of the reservoir (2), the fluid line (4) or the cannula assembly (3) towards the at least one electronic probe (8).

4. Medical infusion device (1) according to any one of claims 1 to 3, **characterized in that** the medical infusion device (1) comprises at least one drain chamber (11) that is configured for collecting liquid medicament that is leaking out of at least one of the reservoir (2), fluid line (4) or cannula assembly (3), wherein the drain chamber (11) comprises the at least one electronic probe (8) of the leakage determining module (7).

5. Medical infusion device (1) according to any one of claims 1 to 4, **characterized in that** the leakage determining module (7) comprises at least one electronic reference probe (12) that is configured and arranged for receiving odor molecules and/or flavor molecules of the liquid medicament outside of the medical infusion device (1) and wherein the evaluation electronics (9) is further configured to determine presence of odor and/or flavor of the liquid medicament based on readings of the at least one electronic reference probe (12) and the readings of the at least one electronic probe (8).

6. Medical infusion device (1) according to any one of claims 1 to 5, **characterized in that** the medical infusion device (1) comprises a user interface (13) that is configured to indicate to the user of the medical infusion device (1) presence of a leakage of liquid medicament based on the determination of the leakage determining module (7).

7. Medical infusion device (1) according to any one of claims 1 to 6, **characterized in that** the medical infusion device (1) comprises a disposable portion (1a) and a reusable portion (1b), wherein the disposable portion (1a) comprises at least the reservoir (2), the fluid line (4), the cannula assembly (3) and the at least one electronic probe (8) of the leakage determining module (7) and/or the at least one electronic reference probe (12) of the leakage determining module (7).

8. Medical infusion device (1) according to claim 7, **characterized in that** the reusable portion (1b) comprises the evaluation electronics (9).

9. Medical infusion device (1) according to claim 7, **characterized in that** the disposable portion (1a) comprises the evaluation electronics (9).

10. Medical infusion device (1) according to any one of claims 1 to 9, **characterized in that** the leakage determining module (7) is reversibly separable from the medical infusion device (1).

11. Medical infusion device (1) according to any one of claims 1 to 10, **characterized in that** the evaluation electronics (9) is configured to pause determining a leakage of liquid medicament out of at least one of the reservoir (2), the fluid line (4) or the cannula assembly (3) for a predetermined period of time subsequent to a determination of a leakage of liquid medicament out of at least one of the reservoir (2), the fluid line (4) or the cannula assembly (3).

12. Medical infusion device (1) according to any one of claims 1 to 11, **characterized in that** the at least one electronic probe (8) and/or the at least one electronic reference probe (12) comprises a gas-permeable membrane (13) that is permeable for gaseous odor molecules and/or gaseous flavor molecules of the liquid medicament and impermeable for liquid components of the liquid medicament.

13. Medical infusion device (1) according to any one of claims 1 to 12, **characterized in that** the medical infusion device (1) is a body-wearable medical infusion pump, preferably a body-wearable medical patch pump.

14. Medical infusion system comprising a medical infusion device and a remote control (17), wherein the medical infusion device comprises a reservoir (2) that is configured for holding a liquid medicament, a cannula assembly (3), a fluid line (4) that is in fluid communication with the reservoir (2) and the cannula assembly (3), a drive mechanism (5), and a dispensing member (6) that when being actuated by the drive mechanism (5) dispenses liquid medicament out of the reservoir (2) through the fluid line (4) and the cannula assembly (3), wherein the medical infusion device is operatively coupled to the remote control (17), **characterized in that**
the remote control (17) comprises a leakage determining module (7) that is configured to determine leakage of the liquid medicament out of at least one of the reservoir (2), the fluid line (4) or the cannula assembly (3) of the medical infusion device, wherein the leakage determining module (7) comprises an electronic probe (8) that is configured for receiving odor molecules and/or flavor molecules of the liquid medicament originated from outside the remote control (17), and an evaluation electronics (9) that is configured to determine presence of odor and/or flavor of the liquid medicament based on readings of the at least one electronic probe (8).
